# EUROPEAN PATENT SPECIFICATION

(11) **EP 2 444 114 B1**
(45) Date of publication and mention of the grant of the patent: **28.09.2016**
(21) Application number: 11185927.8
(22) Date of filing: 20.10.2011
(51) Int. Cl.: A61M 16/00, A61M 16/20

(54) **Apparatus for positive expiratory pressure therapy**
Vorrichtung zur positiven Ausatmungsdrucktherapie
Appareil de thérapie de pression expiratoire positive

(30) Priority: 20.10.2010 MY 1004921
(43) Date of publication of application: 25.04.2012
(73) Proprietor: Hill-Rom Services Pte. Ltd., Singapore 768923 (SG)
(72) Inventor: Rozario, Deborah Anne, Leicester, Leicestershire LE12 5HS (GB); Yao, Jee Soo, 544122 Singapore (SG); Kamalashi, Nair Radhakrishnan Oravelil, 822103 Singapore (SG); Guo, Mike Yang Chang, 600232 Singapore (SG)
(74) Representative: Findlay, Alice Rosemary

(56) References cited:
- EP-A1- 1 435 251
- EP-A2- 1 103 287
- WO-A1-2008/063966
- US-A- 5 899 832
- US-A1- 2003 234 017
- US-A1- 2010 101 573

## Description

The present disclosure relates to positive expiratory pressure (PEP) devices, and components thereof.

A PEP device is a medical device that patients use when suffering from lung diseases or secretory problems or other respiratory issues. Such devices provide resistance to exhalation to help the patient remove pulmonary secretions from the lungs and airway. By breathing into the device, pressure is provided which can assist the patient in removing the secretions or mucus, such as by causing the patient to cough, thinning the mucus, and/or getting air behind the mucus. While various PEP devices are known, a need persists in enhancing the features and functionality of such devices, and overcoming various problems or inconveniences associated with such devices.

EP1435251 discloses a respiratory therapy device comprising a mouth portion and a main portion. The mouth portion comprises an air inlet and is sized to be placed in a patient's mouth. The main portion is in fluid communication with the mouth portion, and defines a first airway chamber and a second airway chamber. The first airway chamber comprises a first outlet, and is configured to provide substantially constant resistance to the flow of respiratory air between the air inlet and the outlet. The second airway chamber comprises a second outlet and a moving mechanism configured to provide oscillating resistance to the flow of respiratory air between the air inlet and the second outlet. A selector is provided which is configured to close either the first airway chamber or the second airway chamber from the flow of air, while keeping the other open.

The invention provides a respiratory therapy device which is characterized in that the device further comprises a resistance selector configured to adjust the resistance provided by the first chamber.

In some embodiments, oscillatory PEP is provided via a moving mechanism comprising a rocker arm pivotable about a pivot. A conical shaped plug is connected to the rocker arm to vary engagement with a conical shaped surface defining a portion of the second airway chamber during pivoting of the rocker arm. In other embodiments, oscillatory PEP therapy is provided via a flexible tube, or via a rotating wheel.

In one embodiment, the mouth portion and the main portion are constructed of microwavable materials. It is preferred that all components of the device comprise microwavable materials, such as plastics or other non-metallic materials.

A method of cleaning a respiratory device is disclosed. The method comprises providing a respiratory device. The device includes a mouth portion sized to be placed in a patient's mouth and configured to receive air, and a main portion in fluid communication with the mouth portion and providing a resistance to the flow of air. The method further comprises placing the device in a microwave oven, and running the microwave oven at a power and for a time that provides heating of the device sufficient to substantially sterilize the device.

Still other embodiments can include any one or more of the following features, alone or in any combination: 1) a combination of oscillation PEP therapy components and standard PEP therapy components in a single PEP device, 2) microwavable materials, 3) an oscillation rocker that produces a venturi effect, 4) a flexible tube to create air pressure oscillation, 5) a rotating wheel to open and close the air channel and create air pressure oscillation, 6) a variable cross section air channel to generate different air flow resistance, 7) a flexible air flow stopper plate with adjustable pivot point to generate different air flow resistance, and/or 8) a selection switch that allows the device to switch between standard PEP therapy and oscillatory PEP therapy.

The invention will now be further described by way of example with reference to the accompanying drawings, in which:
FIG. 1 is a block diagram showing one embodiment having both oscillatory PEP and constant PEP therapy components;
FIG. 2 is a schematic side view of an embodiment having both oscillatory PEP and constant PEP chambers, and the ability to switch between the two;
FIG. 3 is schematic diagram illustrating examples of rocker arm and venturi plug components which can be used to carry out oscillatory PEP therapy, and which can be used with the embodiments of FIGS. 1 or 2 or other embodiments;
FIG. 4 is schematic diagram illustrating an example of a flexible tube system which can be used to carry out oscillatory PEP therapy, and which can be used with the embodiments of FIGS. 1 or 2 or other embodiments;
FIG. 5 is schematic diagram illustrating an example of a rotating wheel system which can be used to carry out oscillatory PEP therapy, and which can be used with the embodiments of FIGS. 1 or 2 or other embodiments;
FIG. 6 is a side view of an example of a knob with adjustable opening, which can be used to adjust the resistance provided by constant pressure PEP therapy components, and which can be used with the embodiments of FIGS. 1 or 2 or other embodiments;
FIG. 7 is a schematic side view of an example of a flexible air flow stopper that has a movable pivot point, which can be used to adjust the resistance provided by constant pressure PEP therapy components, and can be used with the embodiments of FIGS. 1 or 2 or other embodiments;
FIG. 8 is a schematic side view of an example of a plate having multiple sized openings which can be used to adjust the resistance provided by constant pressure PEP therapy components, and can be used with the embodiments of FIGS. 1 or 2 or other embodiments;
FIG. 9 is schematic side view of an example of a tube having selectable openings, which can be used to adjust the resistance provided by constant pressure PEP therapy components, and can be used with the embodiments of FIGS. 1 or 2 or other embodiments;
FIGS. 10a-c are side, front and back views of an example of a PEP device that may include one or more of the features described herein;
FIGS. 11 a-g are various views of an example of PEP device that may include one or more of the features described herein;
FIGS. 12a-c are perspective, front, and back views of an example of PEP device that may include one or more of the features described herein; and
FIGS. 13a-g are various views of an example of PEP device that may include one or more of the features described herein.

Many other embodiments are also possible.

One embodiment of the present disclosure is a PEP therapy device that provides multiple PEP therapy types and ability to switch between these types, uses cleanable materials, uses a venturi effect, and uses particular components to create variability of the air flow resistance. Other embodiments can include any one or more of the following features, alone or in any combination: 1) a combination of oscillation PEP therapy and standard PEP therapy components, 2) use of microwavable materials, 3) use of an oscillation rocker that produces a venturi effect, 4) use of a flexible tube to create air pressure oscillation, 5) use of a rotating wheel to open and close the air channel to vary resistance, 6) use of a variable cross section air channel to generate different air flow resistance, 7) use of a flexible air flow stopper plate with adjustable pivot point to generate different air flow resistance, and/or 8) use of a selection switch that allows the device to switch between standard PEP therapy and oscillatory PEP therapy. These and/or other additional features may comprise patentable subject matter and will become apparent to those skilled in the art upon consideration of the following detailed description of various embodiments exemplifying the best mode of carrying out the embodiments as presently perceived.

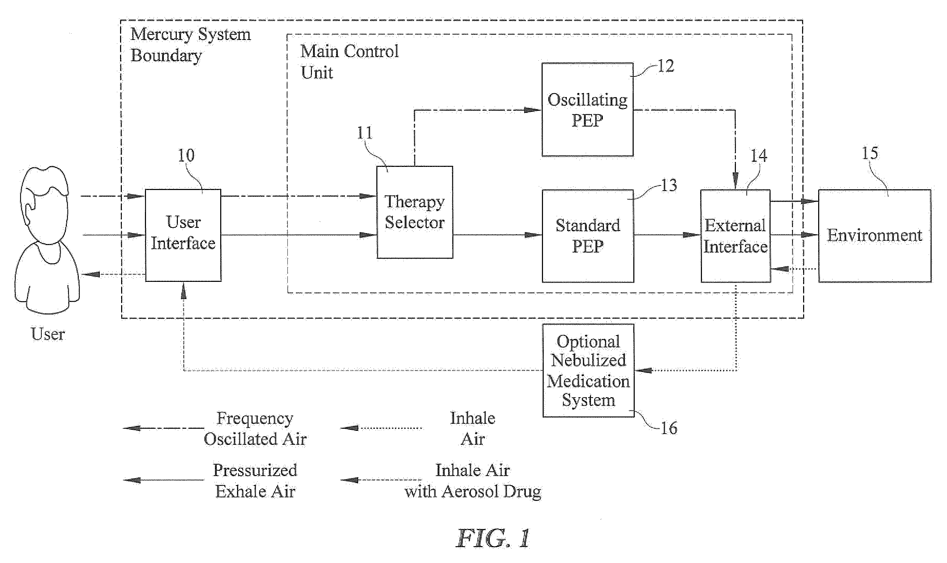

FIG. 1 is a block diagram showing one embodiment of a system having both oscillatory PEP and constant PEP therapy components. In this embodiment, a user interface 10 is provided to allow the patient to breathe into the system. A therapy selector 11 allows the user to select the desired therapy, such as by allowing air to flow through either one of the oscillating PEP components 12 or the constant pressure PEP components 13. External interface components 14, which may include an outlet, allows the system to connect to the environment or ambient air. In addition, the external interface components 14 allow the system to connect to a medication delivery system. For example, the components 14 may include a connector that connects to a nebulizer.

In operation, the patient breathes through the interface 10. Air is channeled by the selector components 11 to the selected therapy components (oscillatory or constant pressure, 12 or 13). Air exits the interface 14. If oscillatory therapy is selected, a variable pressure will be provided against the patient's breathing. If standard therapy is selected, a constant pressure will be provided against the patient's breathing. If the medication delivery system 16 is connected, medication can be delivered simultaneously to the patient during inhalation cycles, through the device. The pressure provided by the system of FIG. 1 during exhalation assists the patient in removing secretions such as mucus from the respiratory system, such as by inducing a cough, thinning the mucous, generating vibrations in the airway, and/or getting air behind the mucus.

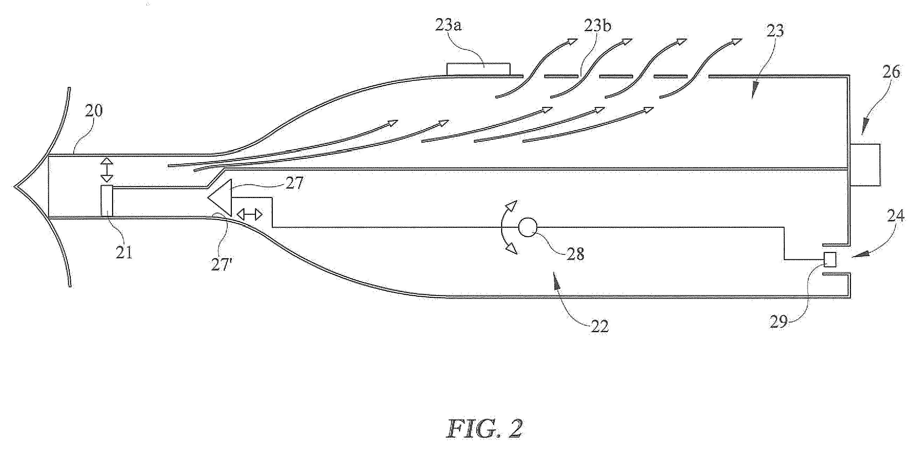

FIG. 2 is a schematic side view of an embodiment having both oscillatory PEP and constant PEP chambers, and the ability to switch between the two. In this example, the device includes a mouth portion having a mouthpiece 20 sized to be placed in the patient's lips. A selector 21 is movable to block either the standard (constant pressure) chamber 23 or the oscillatory chamber 22. Accordingly, either constant pressure PEP therapy or oscillatory PEP therapy can be delivered. If oscillatory PEP is selected, the selector 21 blocks off the standard chamber 23 from the patient, and the patient breathes through oscillatory chamber 22. The components of chamber 22 provide a variable pressure resistance against the flow of breath from the patient during operation. In this example, this is achieved by a rocker arm assembly having conical shaped plug 27 (e.g., with a conical surface) that engages a conical surface 27' of the chamber 22. The rocker arm rotates about a pivot point 28 as the patient expels breath through the device. A venturi effect, caused by movement of the conical shaped plug 27, moves the plug 27 back and forth (decreases in pressure at the conical surfaces cause the plug to be pulled in, while the patient's breath cause the plug to be pushed back out). This causes pivoting motion of the rocker arm assembly about the pivot point 28. Oscillatory pressure resistance results. The air is expelled out of the outlet 24, which is closed and opened via a plug 29.

If standard PEP is selected, the selector 21 blocks off the oscillatory chamber 22 from the patient, and the patient breathes through standard chamber 23. The components of chamber 23 provide an approximately constant pressure resistance against the flow of breath from the patient. In this example, this is achieved by one or more openings 23b that allow the breath to flow out of the chamber 23. A selector 23a may be provided to allow the number of openings, and thus the constant resistance, to be adjusted by the user. The selector 23a in this example is a slide that covers one or more openings 23b. This example also includes a connector 26 for connecting to a medication delivery system, to allow the patient to receive medication during the breathing therapy.

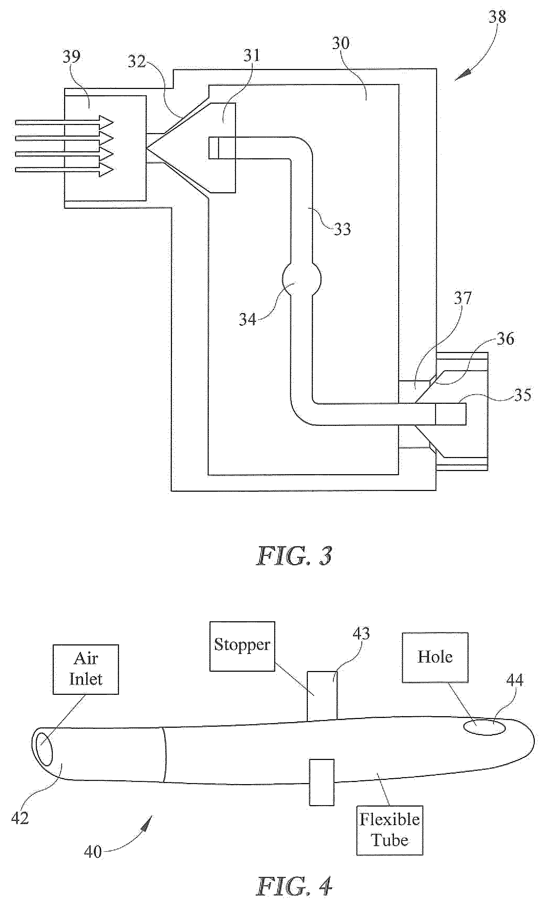

FIG. 3 is schematic diagram illustrating examples of rocker arm and venturi plug components which can be used to carry out oscillatory PEP therapy, and which can be used with the embodiments of FIGS. 1 or 2 or other embodiments. Here, the oscillatory system 38 comprises a plug 31 with a conical shaped surface. The plug 31 engages a similarly shaped surface defined by the housing of the system 38. The plug 31 is movable via a rocker arm 33 that is pivotable about pivot point 34. A plug 35 having a seal 36 partially seals outlet 37. As the patient breathes out, air moves in through inlet 39, through chamber 30, and out outlet 37. The force of the air pushes plug 31, causing rocker arm 33 to pivot one way, but the shape of the plug 31 creates a venturi effect that then reduces pressure causing it to pull back in toward the inlet 39 and causing rocker arm 33 to then pivot back the other way. This back and forth motion provides variable resistance against the patient's breath.

FIG. 4 is schematic diagram illustrating an example of a flexible tube system which can be used to carry out oscillatory PEP therapy, and which can be used with the embodiments of FIGS. 1 or 2 or other embodiments. In this example, a flexible tube 40 is provided that includes a stopper 43, an air inlet 42, and an outlet hole 44. When the patient's breath flows through the tube 40, the latter half of the tube oscillates, or shakes due to the flexible nature of the tube. Modifying the location of the stopper 43 along the tube provides a modification to the amount the latter half of the tube can oscillate during breathing. Accordingly, the frequency of oscillation is modified.

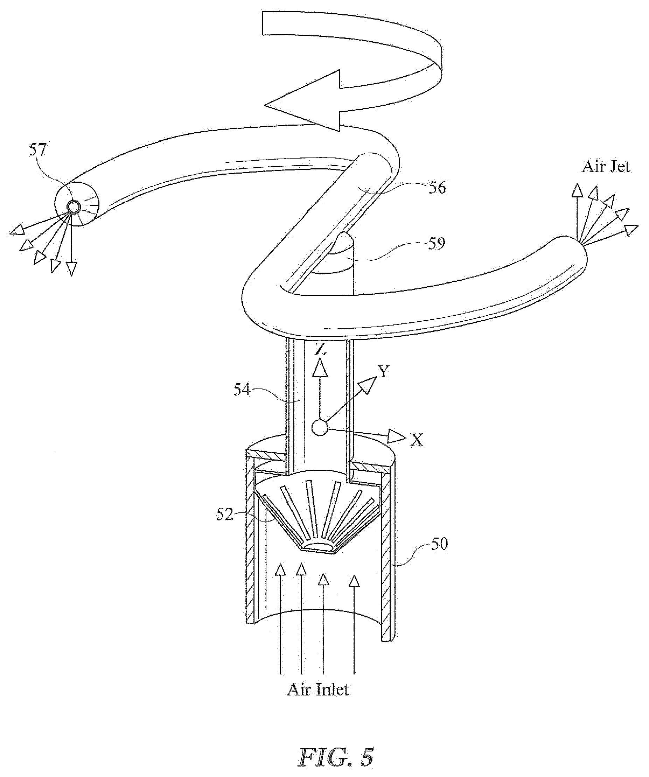

FIG. 5 is schematic diagram illustrating an example of a rotating wheel system which can be used to carry out oscillatory PEP therapy, and which can be used with the embodiments of FIGS. 1 or 2 or other embodiments. The system includes an air inlet 50, a conical diffuser 52, and a flow tube 54. In addition, the system includes a flexible curved wheel tube 56 that rotates about a pivot 59. The patient's breath flows through the air inlet and diffuser 52 and the air tube 54, and flows out the two ends of the rotating wheel tube 56. The diffuser 52 and tube 56 create a variable resistance to the flow of the air.

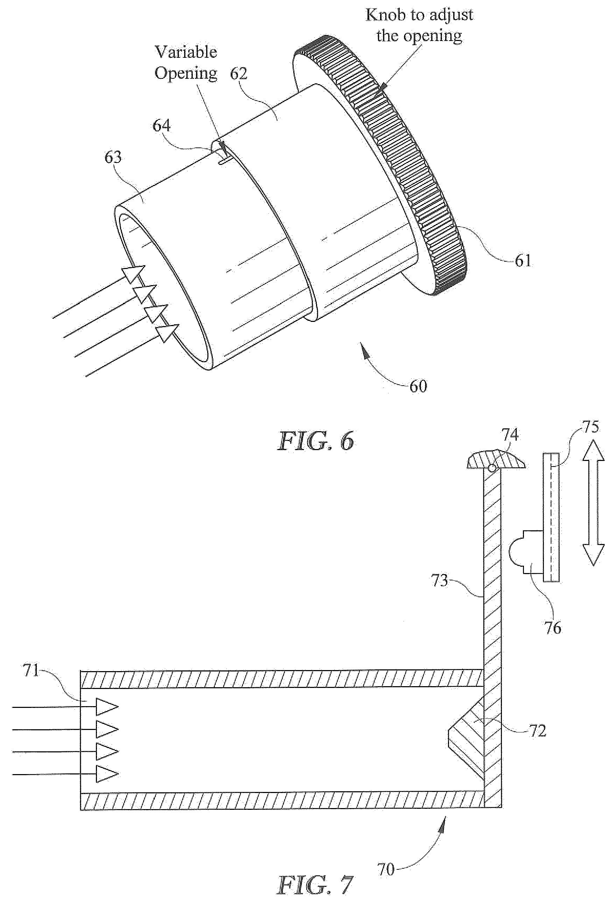

FIG. 6 is a side view of an example of a knob with adjustable opening, which can be used to adjust the resistance provided by constant pressure PEP therapy components, and which can be used with the embodiments of FIGS. 1 or 2 or other embodiments. In this example, a knob 61 is used to set the level of constant pressure provided by the constant pressure PEP system. The knob adjusts the amount of telescope between the tube 62 relative to the tube 63. This adjustment allows the variable opening 64 to be more open or less open. The patient's breath flows in the tube 63 and out the opening 64. The size of the opening 64 controls how much resistance is provided against the patient's breath. Accordingly, the level of resistance can be set by the patient via the knob 61.

FIG. 7 is a schematic side view of an example of a flexible air flow stopper that has a movable pivot point, which can be used to adjust the resistance provided by constant pressure PEP therapy components, and can be used with the embodiments of FIGS. 1 or 2 or other embodiments. In this example, the air flows in the inlet 71 and through the opposite end of the tube 70 around the stopper 72. The stopper is biased toward the tube 70 but can pivot away from the tube 70 under force of the air flow. The amount of force or resistance that the stopper 72 provides against the flow is adjusted by way of the movable pivot 76. The movable pivot 76 can be placed closer to the stopper 72 to increase the force and resistance, or further from the stopper 72 to decrease the force and resistance. Accordingly, the patient can adjust the level of airway resistance provided by adjustment of the pivot 76, which is movable via a knob, sliding button, or similar selector schematically illustrated in 75.

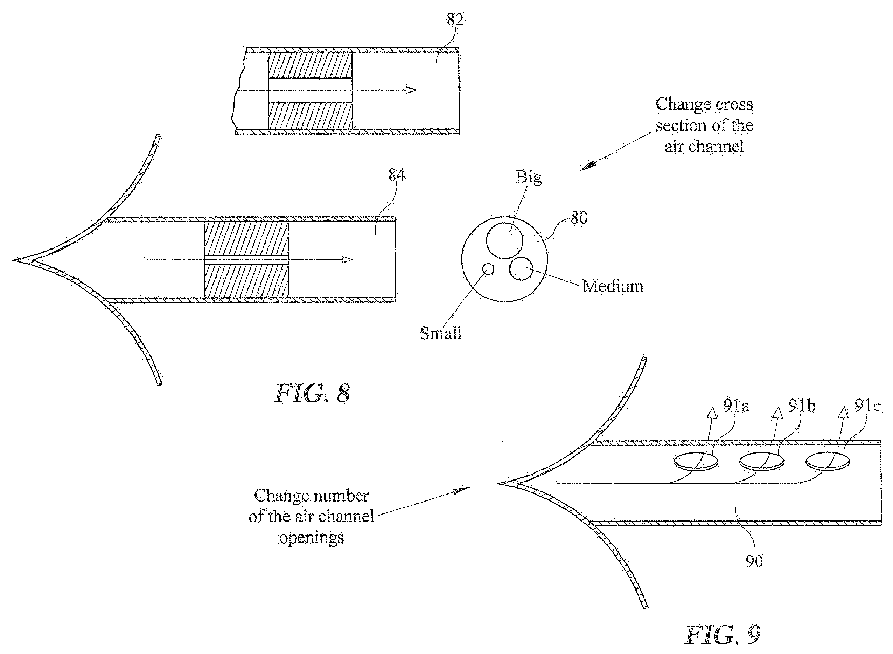

FIG. 8 is a schematic side view of an example of a plate having multiple sized openings which can be used to adjust the resistance provided by constant pressure PEP therapy components, and can be used with the embodiments of FIGS. 1 or 2 or other embodiments. In this example, multiple sized openings are provided on a rotatable plate 80. The plate 80 is placed inside tube 82/84. The patient can rotate the plate 80 to align the desired opening with the airflow path. If a large opening is aligned with the airflow path, as shown with tube 82, then the constant resistance is set to a low level. If a small opening is aligned with the airflow path, as shown with tube 84, then the constant resistance is set to a high level. The patient can rotate the selector plate 80 by placing a finger on its edge through an opening in tube 82/84 and rotating it to the desired position.

FIG. 9 is schematic side view of an example of a tube having selectable openings, which can be used to adjust the resistance provided by constant pressure PEP therapy components, and can be used with the embodiments of FIGS. 1 or 2 or other embodiments. Here, the tube 90 has multiple openings 91a-c. The patient can cover or uncover one or more openings 91a-c, such as via a sliding selector cover. The level of resistance is thus set by the number of openings that are covered.

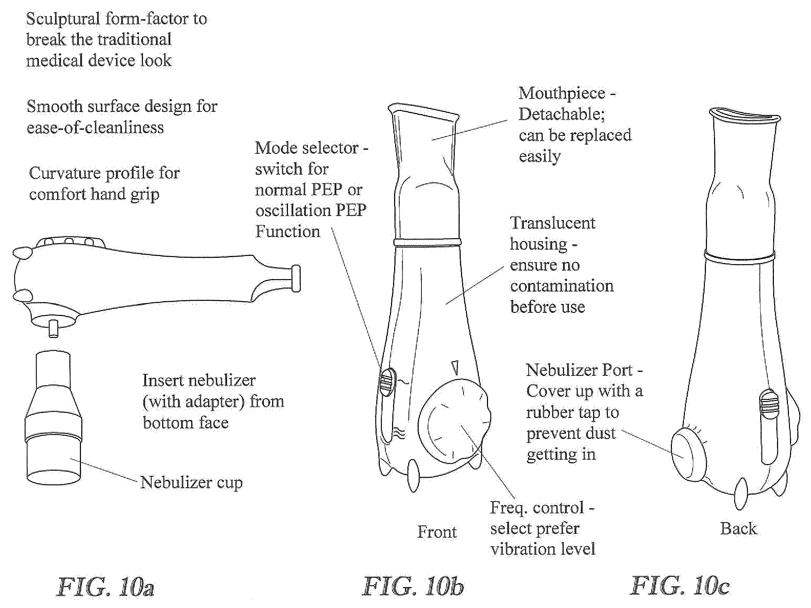

FIGS. 10a-c are side, front and back views of an example of a PEP device that may include one or more of the features described herein. In this example, a nebulizer cup connects to one end of the device. A selector switch on the side allows the user to select between oscillatory PEP or constant pressure PEP. The oscillation level can also be adjusted via a rotary selector knob. The knob could adjust a pivot point, as described above.

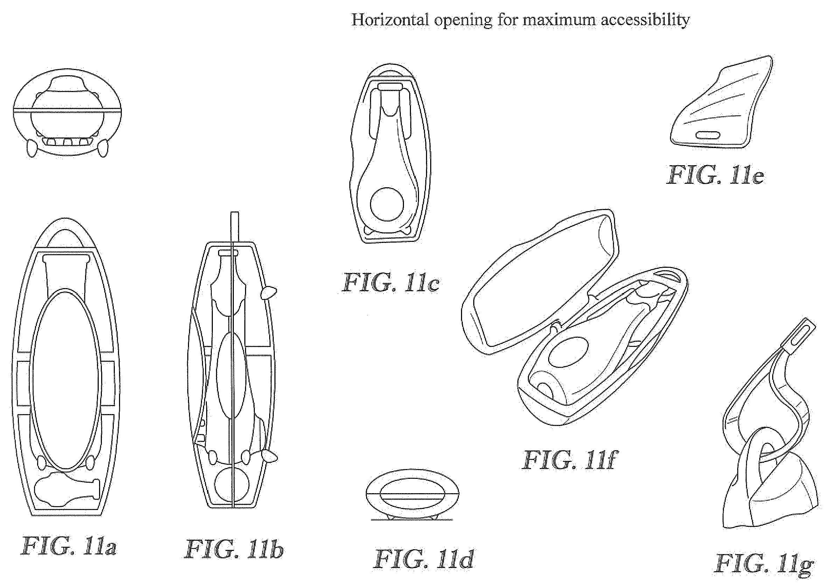

FIGS. 11a-g are various views of an example of PEP device that may include one or more of the features described herein. Here, a case is provided to protect and store the PEP device. Also, a carrying strap connects to the case for ease of carrying.

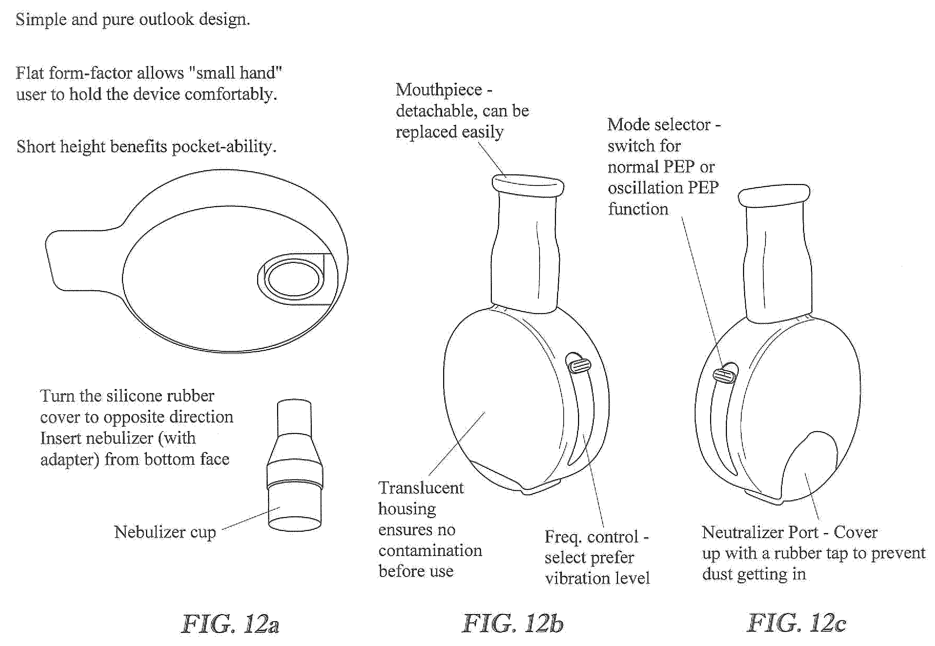

FIGS. 12a-c are perspective, front, and back views of an example of a PEP device that may include one or more of the features described herein. In this example, a detachable mouthpiece is provided for ease of cleaning and replacement when needed. Additionally, a slidable resistance selector switch is provided on one side of the device to allow the oscillation level to be adjusted, and a slidable mode selector switch is provided on the opposite side to allow the device to switch between constant pressure PEP therapy and oscillating PEP therapy. The nebulizer port includes a cover to prevent dust from entering.

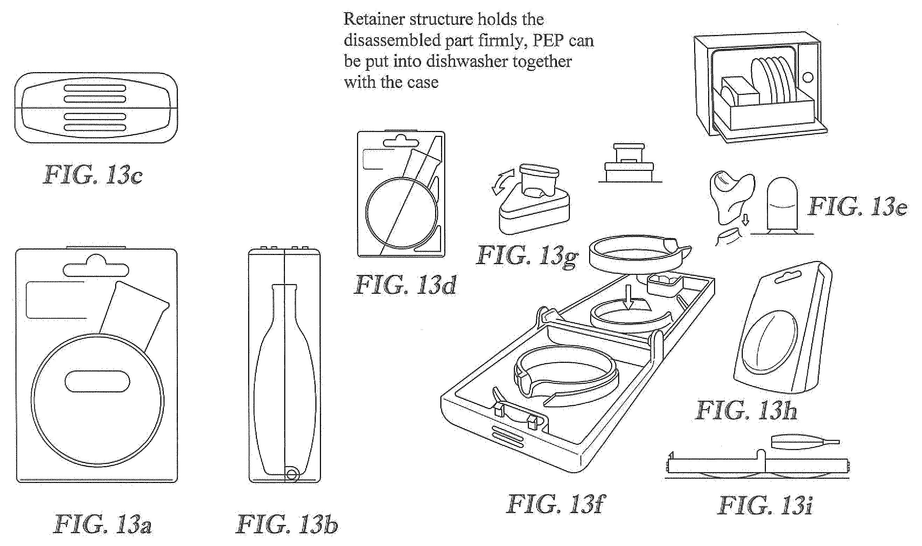

FIGS. 13a-g are various views of an example of PEP device that may include one or more of the features described herein. This embodiment includes a carrying case with retainer structure that holds all components of the device. The case holds the components separately so that they can be exposed to water in a dishwasher, for ease of cleaning and sanitization.

All or substantially of the components of above embodiments can be made of microwavable materials, such as non-metallic materials for example. Plastic could be used for example. Accordingly, to clean the device, the patient can place the device in a microwave oven for sufficient time and at a sufficient microwave power to permit the device to be heated, cleaned, disinfected, and/or sterilized. The heat and/or the microwaves can kill germs and bacteria in or on the components.

Although certain illustrative embodiments have been described in detail above, many other embodiments, variations, and modifications are possible. For example, while oscillatory pressure resistance is described, it is contemplated that such components may otherwise provide oscillation to the airway, whether through pressure or otherwise, and whether or not the oscillation is sinusoidal, periodic, or otherwise changing back and forth.

## Claims

1. A respiratory therapy device, comprising a mouth portion (20) comprising an air inlet (39, 42, 50, 71) and sized to be placed adjacent a patient's lips, and a main portion in fluid communication with the mouth portion, and defining a first airway chamber (23) and a second airway chamber (22) and comprising a chamber selector (21) configured to close one of the first airway chamber (23) and second airway chamber (22) from the flow of air, while keeping the other open; wherein the first airway chamber (23) comprises a first outlet (23b, 64, 91 a-c), and is configured to provide substantially steady resistance to the flow of respiratory air between the air inlet (39, 42, 50, 71) and the first outlet, and wherein the second airway chamber (22) comprises a second outlet (24, 37, 44) and an oscillator configured to provide oscillating resistance to the flow of respiratory air between the air inlet (39, 42, 50, 71) and the second outlet (24, 37, 44), **characterized in that** the device further comprises a resistance selector (23a, 61, 75) configured to adjust the resistance provided by the first chamber (23).

2. The device as recited in either claim 1, wherein the first outlet (23b, 64, 91a-c) of the first airway chamber (23) is adjustable in size via the resistance selector (23a, 61, 75).

3. The device as recited in any preceding claim, wherein the first airway chamber includes a stopper (72) that provides selectable resistance to the flow of air via a movable pivot (76) that is movable via the resistance selector (75).

4. The device as recited in claim 1 or 2, wherein the first airway chamber includes a movable plate (80) and wherein the movable plate (80) includes outlets of varying size which are selected via the resistance selector to act as the first outlet.

5. The device as recited in any preceding claim, wherein the oscillator comprises a moving mechanism comprising a rocker arm (33) pivotable about a pivot, and a conical shaped plug (27, 31) connected to the rocker arm (33) to vary engagement with a conical shaped surface (27') defining a portion of the second airway chamber (22) during pivoting of the rocker arm (33).

6. The device as recited in any one of claims 1 to 4, wherein the moving mechanism comprises a flexible tube (40, 56).

7. The device of any one of claims 1 to 4 wherein the second airway chamber (22) has a nonconstant cross-section area that provides a venturi effect to airflow, thereby creating oscillating resistance to the flow of air.

8. The device as recited in claim 7, wherein the second airway chamber includes a plug (35) that moves in response to the venturi effect.

9. The device as recited in claim 8 wherein the plug (35) is connected to a pivoting rocker arm (33).

10. The device as recited in any one of claims 1 to 4 wherein the oscillator comprises a flexible curved wheel tube (56).

11. The device of any preceding claim wherein the mouth portion (20) and the main portion are constructed of microwavable materials.

12. The device as recited in claim 11, wherein all components of the device comprise microwavable materials.

13. The device of any one of claims 1 to 10 wherein the mouth portion (20) and the main portion are constructed of nonmetallic materials and wherein the device includes no metallic materials.

## Patentansprüche

1. Atemtherapiegerät, bestehend aus einem Mundstück (20) mit einem Luftzuführungseingang (39, 42, 50, 71), das so dimensioniert ist, dass es im Bereich der Lippen eines Patienten aufgesetzt werden kann, und einem Hauptteil, das zum Durchgang eines Fluids mit dem Mundstück in Verbindung steht und eine erste Luftkanalkammer (23) und eine zweite Luftkanalkammer (22) bildet und eine Kammerwählvorrichtung (21) umfasst, die so konfiguriert ist, dass entweder die erste Luftkanalkammer (23) oder die zweite Luftkanalkammer (23) geschlossen und ein Luftdurchgang unterbunden wird, wobei der jeweils andere Kanal offen bleibt; wobei die erste Luftkanalkammer (23) einen ersten Ausgang (23b, 64, 91a-c) umfasst und so konfiguriert ist, dass ein weitgehend konstanter Widerstand gegenüber dem Durchgang von Atemluft zwischen dem Luftzuführungseingang (39, 42, 50, 71) und dem ersten Ausgang gegeben ist, und wobei die zweite Luftkanalkammer (22) einen zweiten Ausgang (24, 37, 44) und einen Oszillator umfasst, der so konfiguriert ist, dass sich ein schwingender Widerstand gegenüber dem Durchgang von Atemluft zwischen dem Luftzuführungseingang (39, 42, 50, 71) und dem zweiten Ausgang (24, 37, 44) ergibt , **dadurch gekennzeichnet, dass** zum Gerät des Weiteren ein Widerstandswähler (23a, 61, 75) gehört, der konfiguriert ist, um den durch die erste Kammer (23) gegebenen Widerstand einzustellen.

2. Gerät nach Anspruch 1, wobei der erste Ausgang (23b, 64, 91 a-c) der ersten Luftkanalkammer (23) über den Widerstandswähler (23a, 61, 75) größenmäßig verändert werden kann.

3. Gerät nach irgendeinem der vorhergehenden Ansprüche, wobei die erste Luft-kanalkammer einen Stopper (72) umfasst, mit dem der Widerstand gegenüber dem Luftdurchgang über einen beweglichen Zapfen (76) einstellbar ist, der über den Widerstandswähler (75) bewegt werden kann.

4. Gerät nach Anspruch 1 oder Anspruch 2, wobei die erste Luftkanalkammer eine bewegliche Platte (80) besitzt und wobei die bewegliche Platte (80) mit Ausgängen unterschiedliher Größe versehen ist, die jeweils als erster Ausgang über den Widerstandswähler angewählt werden können.

5. Gerät nach irgendeinem der vorhergehenden Ansprüche, wobei der Oszillator einen Bewegungsmechanismus mit einem um einen Zapfen schwingenden Kniehebel (33) und einen Stopfen (27, 31) von konischer Form umfasst, der mit dem Kniehebel (33) verbunden ist, um den Eingriff mit einer konisch geformten Fläche (27) zu verändern, die während des Schwingens des Kniehebels (33) einen Teil der zweiten Luftkanalkammer (22) bildet.

6. Gerät nach irgendeinem der Ansprüche 1 bis 4, wobei zum Bewegungsmechanismus ein flexibler Schlauch (40, 56) gehört.

7. Gerät nach irgendeinem der Anspruche 1 bis 4, wobei die zweite Luftkanalkammer (22) eine unregelmäßige Querschnittfläche hat, durch die sich für die Luftströmung ein Venturi-Effekt ergibt und dadurch ein schwingender Widerstand gegen den Luftdurchgang entsteht.

8. Gerät nach Anspruch 7, wobei die zweite Luftkanalkammer einen Stopfen (35) umfasst, der sich in Abhängigkeit vom Venturi-Effekt bewegt.

9. Gerät nach Anspruch 8, wobei der Stopfen (35) mit einem schwingenden Kniehebel (33) verbunden ist.

10. Gerät nach irgendeinem der vorhergehenden Ansprüche 1 bis 4, wobei der Oszillator einen flexiblen Kurvenaradschlauch (56) umfasst.

11. Gerät nach irgendeinem der vorhergehenden Ansprüche, wobei das Mundstück (20) und das Hauptteil aus mikrowellenfähigen Materialien bestehen.

12. Gerät nach Anspruch 11, wobei für alle Bauteile des Geräts mikrowellenfähige Materialien verwendet werden.

13. Gerät nach irgendeinem der Ansprüche 1 bis 10, wobei das Mundstück (20) und das Hauptteil aus nichtmetallischen Materialien gefertigt sind und wobei im Gerät keinerlei metallische Materialien zum Einsatz kommen.

## Revendications

1. Dispositif de thérapie respiratoire, comprenant une partie de bouche (20) comprenant une entrée d'air (39, 42, 50, 71) et dimensionnée pour être placée de manière adjacente aux lèvres d'un patient, et une partie principale en communication fluidique avec la partie de bouche, et définissant une première chambre de voie aérienne (23) et une seconde chambre de voie aérienne (22) et comprenant un sélecteur de chambre (21) configuré pour fermer l'une de la première chambre de voie aérienne (23) et de la seconde chambre de voie aérienne (22) au flux d'air, tout en maintenant l'autre ouverte ; dans lequel la première chambre de voie aérienne (23) comprend une première sortie (23b, 64, 91a-c) et est configurée pour fournir une résistance sensiblement régulière au flux d'air respiratoire entre l'entrée d'air (39, 42, 50, 71) et la première sortie, et dans lequel la seconde chambre de voie aérienne (22) comprend une seconde sortie (24, 37, 44) et un oscillateur configuré pour fournir une résistance oscillante au flux d'air respiratoire entre l'entrée d'air (39, 42, 50, 71) et la seconde sortie (24, 37, 44), **caractérisé en ce que** le dispositif comprend en outre un sélecteur de résistance (23a, 61, 75) configuré pour ajuster la résistance fournie par la première chambre (23).

2. Dispositif selon la revendication 1, dans lequel la première sortie (23b, 64, 91a-c) de la première chambre de voie aérienne (23) est ajustable en taille via le sélecteur de résistance (23a, 61, 75).

3. Dispositif selon l'une quelconque des revendications précédentes, dans lequel la première chambre de voie aérienne comprend une butée (72) qui fournit une résistance sélectionnable au flux d'air via un pivot mobile (76) qui est mobile via le sélecteur de résistance (75).

4. Dispositif selon la revendication 1 ou 2, dans lequel la première chambre de voie aérienne inclut une plaque mobile (80) et dans lequel la plaque mobile (80) inclut des sorties de taille variable qui sont sélectionnées via le sélecteur de résistance pour agir comme la première sortie.

5. Dispositif selon l'une quelconque des revendications précédentes, dans lequel l'oscillateur comprend un mécanisme mobile comprenant un bras à bascule (33) pouvant pivoter autour d'un pivot, et un obturateur de forme conique (27, 31) relié au bras à bascule (33) pour faire varier la mise en prise avec une surface de forme conique (27') définissant une partie de la seconde chambre de voie aérienne (22) lors du pivotement du bras à bascule (33).

6. Dispositif selon l'une quelconque des revendications 1 à 4, dans lequel le mécanisme mobile comprend un tube flexible (40, 56).

7. Dispositif selon l'une quelconque des revendications 1 à 4, dans lequel la seconde chambre de voie aérienne (22) possède une surface transversale non constante qui fournit un effet venturi au flux d'air, créant ainsi une résistance oscillante au flux d'air.

8. Dispositif selon la revendication 7, dans lequel la seconde chambre de voie aérienne inclut un obturateur (35) qui se déplace en réponse à l'effet venturi.

9. Dispositif selon la revendication 8, dans lequel l'obturateur (35) est relié à un bras à bascule pivotant (33).

10. Dispositif selon l'une quelconque des revendications 1 à 4, dans lequel l'oscillateur comprend un tube en roue incurvé flexible (56).

11. Dispositif selon l'une quelconque des revendications précédentes, dans lequel la partie de bouche (20) et la partie principale sont constituées de matières micro-ondables.

12. Dispositif selon la revendication 11, dans lequel tous les composants du dispositif comprennent des matières micro-ondables.

13. Dispositif selon l'une quelconque des revendications 1 à 10, dans lequel la partie de bouche (20) et la partie principale sont constituées de matières non métalliques et dans lequel le dispositif n'inclut aucune matière métallique.
